# EUROPEAN PATENT SPECIFICATION

(11) **EP 1 929 959 B1**
(45) Date of publication and mention of the grant of the patent: **07.04.2010**
(21) Application number: 06024995.0
(22) Date of filing: 04.12.2006
(51) Int. Cl.: A61B 17/02, A61B 1/31, A61M 29/00

(54) **A surgical device for transanally accessing the rectum of a patient**
Chirurgische Vorrichtung für Einfügung im Rektum eines Patienten
Instrument chirurgical insérable par voie rectale

(43) Date of publication of application: 11.06.2008
(73) Proprietor: ETHICON ENDO-SURGERY, INC., Cincinnati, OH 45242-2839 (US)
(72) Inventor: Longo, Antonio, 90134 Palermo (IT); Kuhns, Jesse J., Cincinnati Ohio 45208 (US); D'Arcangelo, Michele, 00142 Roma (IT); Bilotti, Federico, 04011 Aprilia Latina (IT)
(74) Representative: Leihkauf, Steffen Falk

(56) References cited:
- US-A- 395 705
- US-A- 4 690 132
- US-A- 6 083 241
- US-A- 6 142 931

## Description

The invention relates in general to surgical instruments for the transanal therapy of tissue lesions, e.g. of rectal cancers, benign tumors, polyps or solitary ulcers of the rectal mucosa and particularly to a surgical device for enabling a transanal access in order to perform a transanal therapy, e.g. a superficial or full thickness excision of a lesion, by means of a curved cutter stapler (a so called CCS) at a target site remote from the anal verge of a patient.

Known transanal access systems comprise a circular anal dilator, an obturator having a smoothly tapered or ogive shaped tip to facilitate transanal deployment of the circular anal dilator and an anoscope, e.g. a so called purse string suture anoscope (PSA) which is insertable across the circular anal dilator into the rectum. The purse string suture anoscope (PSA) has a shape of a segment of a substantially cylindrical wall covering typically a circumference of about 270° and leaving a full-length window opening corresponding to about 90° of the total circumference of the rectum. The window opening allows the surgeon to visualize and access at least a part of the target site intended to be manipulated, while the anoscope pushes the remaining mucosa back against the rectal wall along a 270° circumference. In the particular case of a purse string suture anoscope (PSA) a suture can be applied to the mucous membrane that protrudes through the anoscope window and by rotating the anoscope, it is possible to complete a purse string suture around the entire anal circumference.

Thanks to the limited size of the anoscope window in the known purse string suture anoscope, the risk of mucosa collapsing into the working channel and limiting both visualization and working space is comparatively low. Nonetheless, such a small anoscope window size is not at all adapted to provide a sufficient access area for a therapy of lesions by stapling and excision of the lesion using a cutting - stapling device.

In case of transanal therapy using such a cutting - stapling device, the anoscope window is desired to provide an access area of at least about 130° to 180° of the anal circumference in order to properly catch the lesion and position it between the jaws of the cutter-stapler.

While such a wide anoscope window is adapted to completely receive the lesion to be treated, it cannot prevent mucosal tissue from falling into the working channel of the anoscope, thereby occluding visualization and working space.

With the known transanal access devices, visualization becomes definitely impaired beyond 6 cm from the anal verge resulting in the surgeon's view being limited and altered and the working space being limited and, hence, increasing the risk of damaging the rectal wall during surgery.

US 6,142,931 discloses a transanal access device which forms the technical background for the preamble of claim 1.

In view of the drawbacks of the prior art, the object of the invention is to provide a surgical device for transanally accessing the rectum which provides both a good visualization and free working space and a sufficient the access area provided by the anoscope window.

Within the general scope of the main object, it is a further aim of the present invention to provide a surgical device for transanally accessing the rectum having features such as to provide a working space for a cutting - stapling device and, at the same time, to protect the internal sphincter muscle and rectal wall from undesired damage due to the cutter-stapler or other surgical instruments.

It is a yet further aim of the present invention to provide a surgical device for transanally accessing the rectum which enables deeper transanal access to a lesion, particularly up to 10 - 15 cm from the anal verge without the visualization and working space being occluded by rectal wall tissue falling into the working channel of the anoscope.

It is a yet further aim of the present invention to provide a surgical device for transanally accessing the rectum which provides a tissue access area at various depths from the anal verge.

These and other objects are achieved by a surgical device for transanally accessing the rectum according to claim 1 and by a method described below. The dependent claims cover advantageous embodiments of the invention.

According to the invention, the surgical device for transanally accessing the rectum (in the following referred to as "*the device*") comprises the features as disclosed in claim 1 with prefered embodiments in the dependent claims.

In the following descriptions and in the claims, the expressions "distal" and "proximal" refer to the surgeons point of view if not expressly otherwise indicated.

Thanks to the shield member overlapping the window aperture, the tissue access area can be comfortably adapted to the size of the lesion to be treated, e.g. excised by a cutter-stapler and tissue collapse into the working channel between the actual target site and the surgeon is effectively prevented by the combined retaining action of the first and second tissue retaining walls.

In accordance with an aspect of the invention, the shield member is slidable with respect to the anoscope and the second retaining wall is configured to progressively overlap or close the window aperture in response to a progressive sliding movement of the shield member with respect to the anoscope. Particularly, the window aperture extends in a longitudinal (proximal-distal) direction of the anoscope and the shield member is slidable in the same longitudinal (proximal-distal) direction so that the second retaining wall can progressively overlap and close the window aperture from a proximal side thereof. This enables the surgeon to vary the distance of the tissue access area from the anal verge and to prevent tissue from collapsing into the working channel on a proximal side of the lesion which is intended to be accessed by the device, thereby assuring a good visualization and instrumental access to comparatively deep target sites of about 10 to 15 cm from the anal verge.

According to a preferred embodiment, the first retaining wall of the anoscope and the second retaining wall of the shield member are slidingly coupled to each other by longitudinally extending tongue and groove connections of their lateral longitudinal edges. Such a tongue and groove connection assures a good shape-stability of the working channel and obviates the risk of clamping tissue between the anoscope and the shield member.

In accordance with a further embodiment of the invention, the device comprises a long obturator having an elongate substantially cylindrical body with a smoothly rounded, tapered or ogive shaped distal tip. The obturator is removably insertable through the ring wall of the circular anal dilator and through the working channel defined by the anoscope and the shield member so that the rounded distal tip protrudes distally from the anoscope and facilitates the transanal insertion of the entire device.

In accordance with a preferred embodiment, the first retaining wall forms a straight channel having an arched, preferably slightly more than semicircular cross-section (e.g. a half cylinder shell covering an angle of about 180°-230°, preferably about 190°-210°, wherein the expression "half cylinder shell" indicates a generally longitudinally segmented hollow cylinder and not necessarily one halve of a circular cylinder) and defining a longitudinally extending lateral opening forming the anoscope window aperture. Similarly, also the second retaining wall forms a straight channel having an arched, preferably slightly less than semicircular cross-section (e.g. a half cylinder shell covering an angle of about 130°-180°, preferably about 150°-170°) and is arranged inside the anoscope window aperture so that it forms together with the first retaining wall a substantially (preferably but not necessarily circular) cylindrical tube defining internally the working channel for the cutter-stapler and for other surgical instruments. The above indicated non limiting but preferred dimensions of the anoscope and the window aperture make it possible to provide a large enough tissue access area for visualizing and excising a lesion, e.g. a polyp or cancer of the anal wall, while the shield member prevents the mucous membrane or hemorrhoidal tissue disposed on a proximal side (referred to the surgeon) of the tissue access area from falling into the working channel and impairing both the visualization and access to the lesion.

According to a further advantageous embodiment of the invention the anoscope is rotatable and longitudinally slidable with respect to the circular anal dilator, thereby enabling the surgeon to adjust both the circumferential position and the distal distance of the tissue access area from the anal verge according to the specific anatomy of the patient and to the position of the lesion.

In acccordance with a further advantageous embodiment of the invention, the anoscope comprises a distance indicator, preferably a graduated sequence of marks arranged at the internal (i.e. visible) side of the first retaining wall and adapted to provide an immediate visual indication of the depth of insertion of the anoscope, of the distance of the tissue access area from the anal verge, of the relative position between the lesion, a surgical instrument such as a cutter-stapler and the tissue access area.

In order to facilitate transanal insertion of the device, a distal end of the anoscope comprises preferably an arc shaped edge inclined with respect to the longitudinal axis of the anoscope and the distal end of the shield member comprises an arc shaped edge inclined with respect to the longitudinal axis of the shield member which coincides with the longitudinal axis of the anoscope. Advantageously, the arc shaped distal edge of the anoscope and the lateral edges of the anoscope meet at a smoothly rounded obtuse angle in order to prevent tissue damage during insertion of the device. Similarly, the arc shaped distal edge of the shield member and the lateral edge of the anoscope meet preferably at an obtuse angle in order to prevent tissue damage during insertion of the device.

In accordance with a further development of the invention, the anoscope comprises at least one, preferably two recesses formed preferably in two opposite lateral edges of the first retaining wall and adapted to retain and position rectal wall tissue portions for a fast and precise application of a purse string suture or, more generally for providing a reliable reference and tissue support for applying tissue grasping and traction means. Preferably, the two opposite recesses are formed near the distal end of the anoscope and can be circumferentially positioned by turning the anoscope inside the anal dilator and longitudinally positioned by sliding the anoscope inside the anal dilator so that they are available throughout the entire range of access depth of the device.

According to a yet further embodiment of the invention, the above mentioned connecting portion of the circular anal dilator comprises two flat opposite wings protruding laterally outward from the ring wall and inclined towards the proximal side to better adapt to the anatomy of the anus and buttocks of the patient. The wings comprise preferably ring shaped end portions adapted to be sutured to the skin of the buttocks of the patient in order to fixate the position of the device inside the anus.

According to an embodiment, a handle portion protrudes preferably laterally-proximally from the proximal end of the anoscope and is configured to enable manual translational and rotational positioning of the anoscope together with the shield member inside the ring wall of the anal dilator.

These and other details and advantages of the present invention shall be made apparent from the accompanying drawings and the description thereof, which illustrate an embodiment of the invention and, together with the general description of the invention given above, and the detailed description of the invention given below, serve to explain the principles of the present invention.

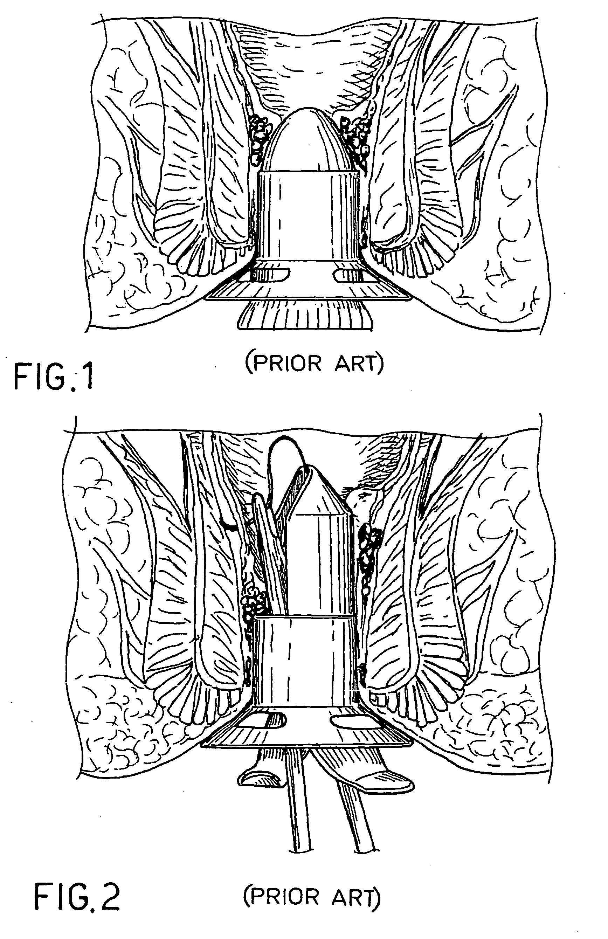

Fig. 1 and 2 are partially sectional views of a prior art transanal access device placed in a patient's rectum;
Fig. 3 is an isometric lateral proximal view of a transanal access device according to an embodiment of the invention;
Fig. 4 is a side view of the device in figure 1 and of a long obturator adapted to facilitate introduction of the device;
Fig. 5 is a further side view of the device in figure 1 and of the long obturator;

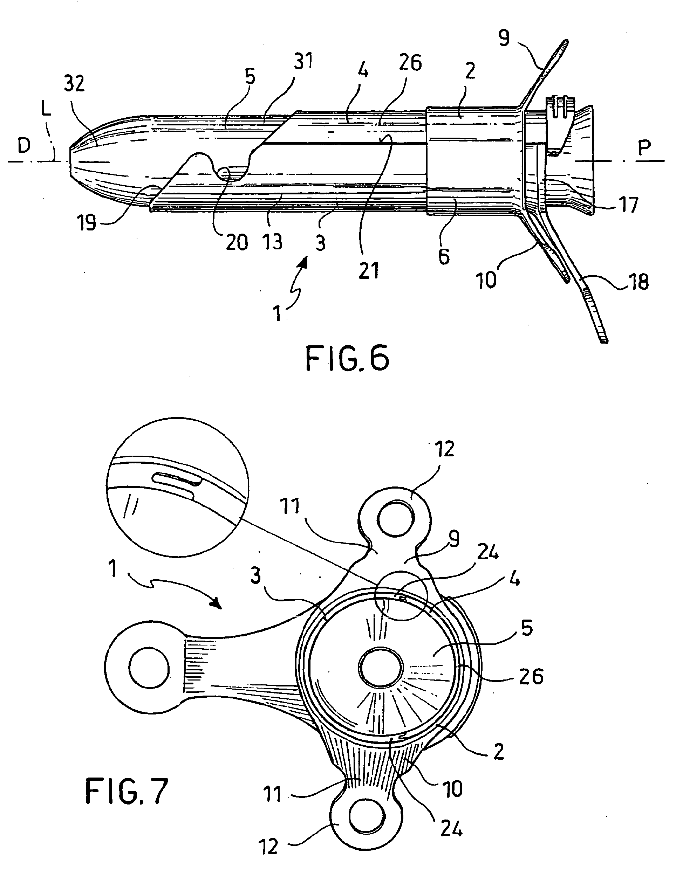

Fig. 6 is a side view of the device in figure 1 assembled with the long obturator to facilitate introduction of the device;
Fig. 7 is a distal end view of the device in figure 6 with an anoscope turned with respect to an anal dilator;

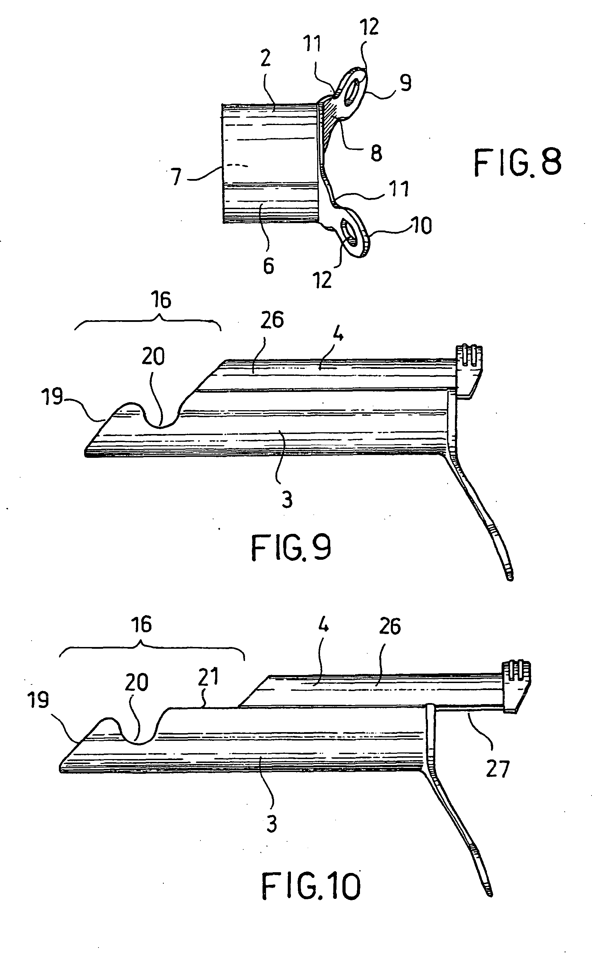

Fig. 8 is a side view of a circular anal dilator of the device in figure 1;
Fig. 9 is a side view of the device in figure 6 with a circular anal dilator and a long obturator removed, in which a shield member is in a first position;
Fig. 10 is the same view of figure 9, in which the shield member is in a second position;

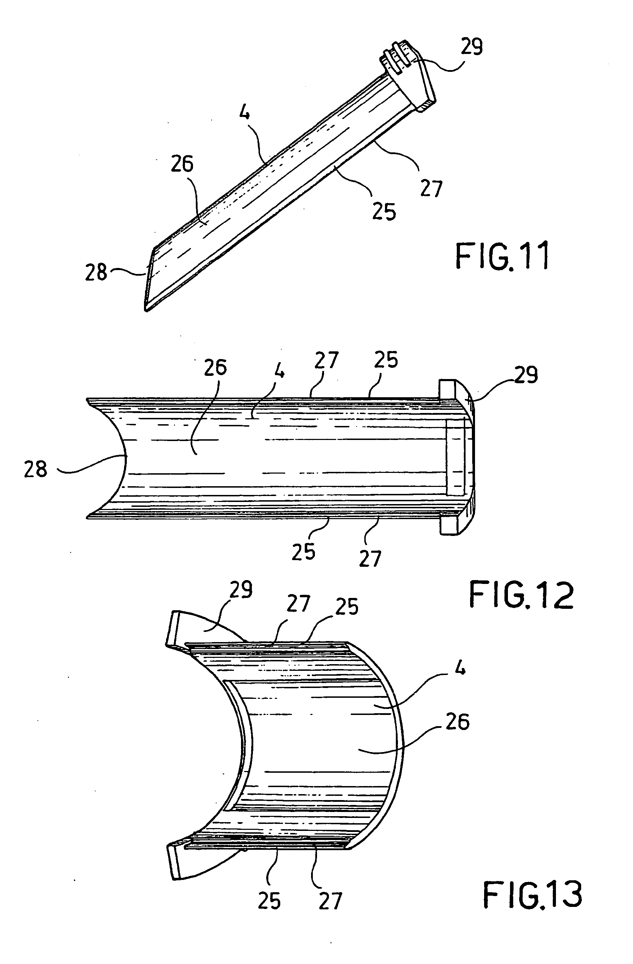

Fig. 11 to 13 are lateral and isometric views of a shield member of the device according to the invention;

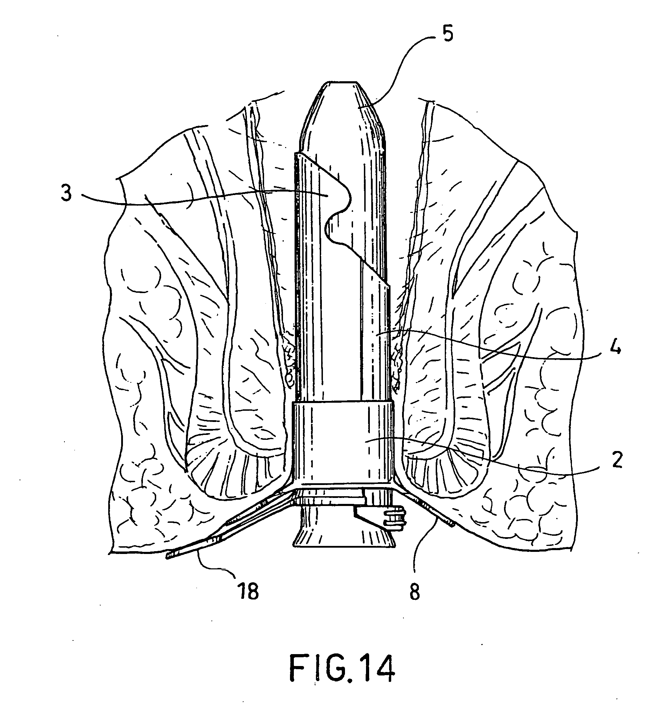

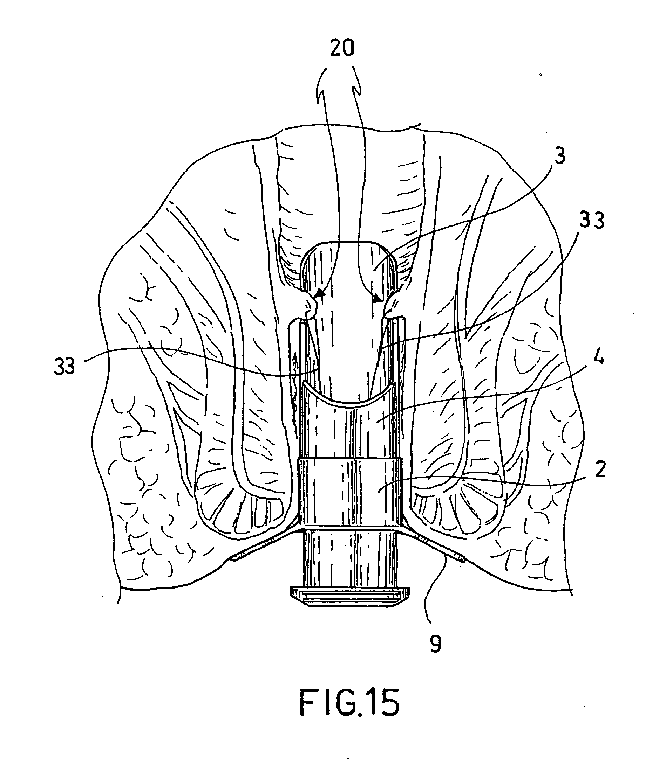

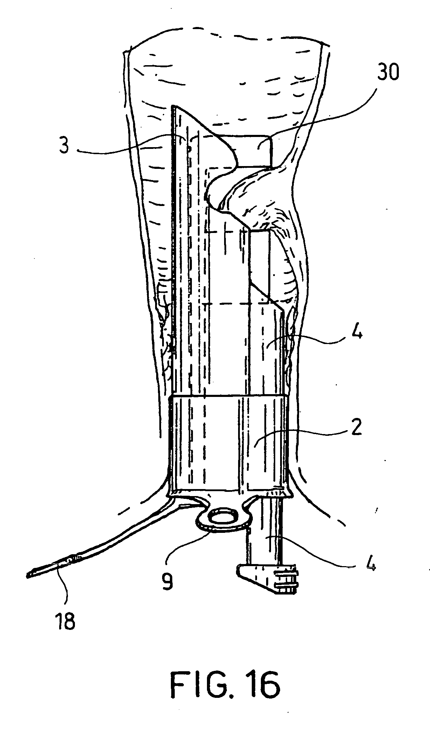

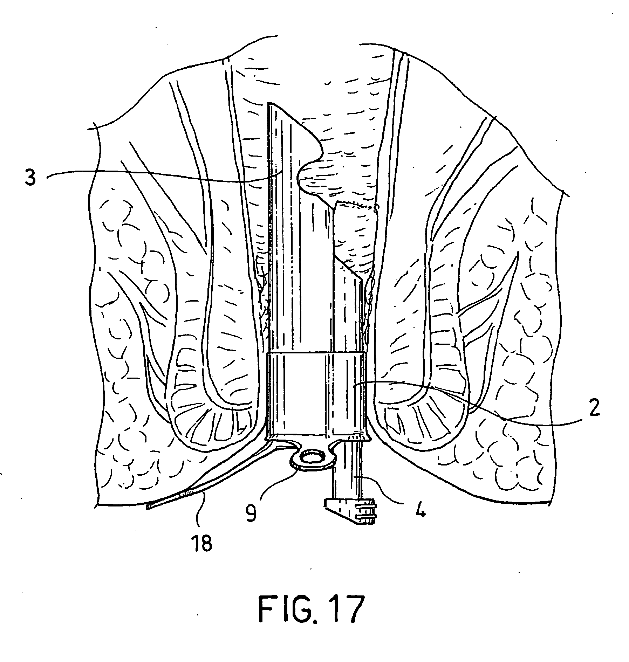

Fig. 14 to 17 illustrate different steps of a method for transanal therapy or transanal full thickness therapy of a lesion of the rectal wall tissue using the transanal access device according to the invention.

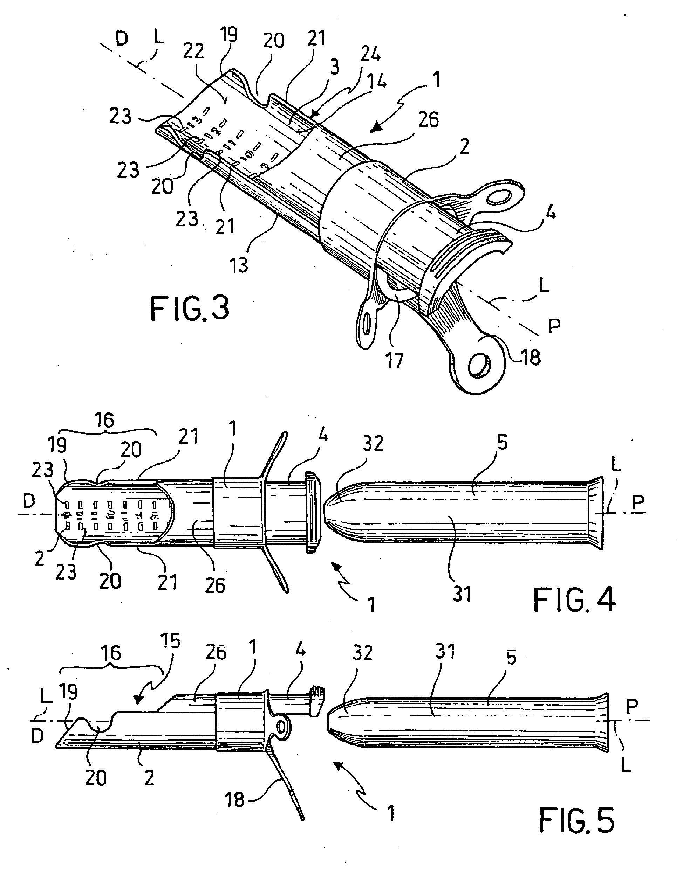

Turning to the figures, fig. 3 is an isometric overall view of a surgical device 1 for transanally accessing the rectum. The device 1 comprises a circular anal dilator 2, an anoscope 3, a shield member 4 and a long obturator 5.

The circular anal dilator 2 (CAD) comprises a circular cylindrical ring wall 6 adapted to be transanally inserted into the rectum and defining internally a passage opening 7 which provides a transanal access opening for visualization and surgery and protects the internal sphincter muscle and the anal wall from damaging. A connecting portion 8 is arranged at a proximal end of the ring wall 6 and adapted to hold the ring wall 6 inside the rectum. The connecting portion 8 comprises two flat opposite wings 9, 10 protruding laterally outward from the ring wall 6 and inclined proximally to adapt to the anatomy of the anus and buttocks of the patient. Each wing 9, 10 forms a narrowed bottleneck section 11 and a ring shaped end portion 12 adapted to be sutured to the skin of the buttocks of the patient in order to fixate the position of the device 1 inside the anus.

The anoscope 3 comprises a first retaining wall 13 having the shape of a longitudinally straight half cylinder shell with an arched cross section and defining at least partially a working channel 14 and a longitudinally extending lateral opening forming a window aperture 15 which provides an access area 16 to the surrounding tissue from inside the working channel 14. The first retaining wall 13 is insertable, longitudinally (in a distal-proximal direction D-P) translatable and rotatable inside the ring wall 6 of the circular anal dilator 2A and comprises a proximal flange 17 which extends radially beyond the internal surface of the ring wall 6 and defines an end of stroke surface which prevents the anoscope 3 from being inserted too far through the ring wall 6 inside the rectum. A flat handle portion 18 protrudes laterally-proximally from the proximal flange 17 and is configured to enable manual rotation and translation of the anoscope 3 inside the ring wall 6 of the anal dilator in order to adjust the position of the access area 16. A distal end of the anoscope 3 or, in other words, of the first retaining wall 13 forms an arc shaped smoothly rounded edge 19 which is inclined with respect to a longitudinal axis L of the anoscope 3, while the cross-section of the first retaining wall 13 maintains its constantly curved half cylindrical shape substantially up to the distal end thereof. Advantageously, the arc shaped distal edge 19 and the lateral edges 21 of the anoscope 3 meet at a smoothly rounded obtuse angle in order to prevent tissue damage during insertion of the device 1.

Near the distal end 19 of the first retaining wall 13, two recesses 20 are formed in the two opposite lateral edges 21 of the first retaining wall 13. The recesses 20 have a rounded substantially halve circle shape and are configured to retain and position rectal wall tissue portions for a fast and precise application of a purse string or traction suture.

At the internal concave surface 22 of the first retaining wall 13, a graduated sequence of marks 23 indicating e.g. a centimeter-scale is printed, embossed or otherwise applied to provide an immediate visual indication of the depth of insertion of the anoscope and of the distance of the tissue access area from the anal verge. Each mark 23 comprises a central numeral indicating a distance from a fixed reference point at the proximal end region of the first retaining wall 13 and one or two reference lines extending on either side of the numeral so that a certain distance can easily be associated also to tissue or instruments which do not lay very close to the numeral without being impaired by the difficult visual prospective provided by the comparatively long tubular working channel 14.

The lateral longitudinal edges 21 of the anoscope 3 are parallel to each other and to the longitudinal axis L and each edge 21 forms a longitudinal groove 24 adapted to slidably receive a corresponding longitudinal tongue 25 formed at the shield member 4 which will be described below.

As already mentioned, the anoscope 3 is slidably and rotatably received by the ring wall 6 of the circular anal dilator 2 and has a length such that it protrudes distally from the ring wall 6 in order to provide the working channel 16 which extends distally to a depth of 10 to 15 cm from the anal verge.

The shield member 4 forms a second retaining wall 26 which is shaped as a longitudinally straight half cylinder shell with an arched cross section. Both lateral longitudinal edges 27 of the second retaining wall 26 are parallel to each other and to the longitudinal axis L and form the above said longitudinally extending tongues 25. The tongues 25 are slidably received by the longitudinal grooves 24 of the first retaining wall 13 so that the shield member 4 is slidably shape-coupled to the anoscope 3 and their first and second retaining walls 13, 26 define together the tubular cylindrical working channel 14 and the tissue access area 16 whose size and position is variable by sliding the shield member 4 longitudinally with respect to the anoscope 3 so that the window aperture 15 is covered more or less by the second retaining wall 26.

The distal end of the shield member 4 forms a rounded arc shaped edge 28 which is inclined with respect to the longitudinal axis of the shield member 4 in a manner that the arc shaped distal edge 28 of the shield member 4 and the lateral edges 21 of the anoscope 3 meet at an obtuse angle in order to prevent tissue from being jammed between these edges 28, 21 during insertion of the device 1 and during proximal sliding of the shield member 4 with respect to the anoscope 3. Similarly to the anoscope 3, also the shield member 4 comprises a proximal flange 29 extending from the proximal end of the second retaining wall 26 radially beyond the passage opening 7 of the circular anal dilator 2, thereby providing a stop surface which prevents the shield member 4 from being pushed completely through the ring wall 6 of the anal dilator 2.

The proximal flange 29 can be directly used as a handle for manually positioning the second retaining wall 26 in order to adapt the size and position of the tissue access area 16 to provide both sufficient tissue access at the target site and sufficient tissue retention around the target size and along the working channel of the device 1.

The long obturator 5 has an elongate substantially cylindrical body 31 with a smoothly rounded, tapered distal tip 32. The obturator 5 is removably insertable through the ring wall 6 of the circular anal dilator 2 and through the working channel 14 defined by the anoscope 3 and the shield member 4 so that the rounded distal tip 32 protrudes distally from the anoscope and facilitates the transanal insertion of the entire device 1. Thanks to the combination of the anoscope and the shield member, the transanal access device 1 can be comparatively long (10 cm to 15 cm) without impairing the viewing and without tissue collapsing into the working channel. Hence, also the obturator 5 needs to be longer than known obturators in order that its distal tip 32 can protrude from the distal end of the anoscope to facilitate its introduction.

The device 1 for transanal access according to the invention makes it possible to perform a method for transanal or full thickness transanal therapy, especially beyond 6 cm from the anal verge by means of a cutter stapler 30, such as the *curved cutter stapler CCS-30* by Ethicon Endo-Surgery, Inc. Such a transanal therapy can involve excision of rectal cancers, benign tumors, polyps or solitary ulcers of the rectal mucosa.

After an initial identification of a suspicious lesion, e.g. via examination of the patient history and or by inspection by sigmoscope, colonoscope, rectoscope, TULIP, etc., the device 1 is completely assembled around the long obturator 5 and introduced into the anus of the patient. The circular anal dilator 2 can now be fixated by suturing its lateral wings 9 extracorporeally to the skin of the patient's buttocks. After introduction of the device 1, the long obturator 5 is removed therefrom to free the working channel 14 defined by the anoscope 3 and the shield member 4. It is now possible to rotate and translate the anoscope 3 alone or together with the shield member 4 inside the circular anal dilator 2 until the lesion appears within the access area 16 defined by the anoscope window aperture 15 so that it becomes viewable by the surgeon. After having identified the position and extension of the lesion through the working channel 14 and anoscope window aperture 15, the surgeon can slide the shield member 4 proximally or distally to adjust the position and dimension of the access area 16 to the position and dimension of the lesion and surrounding tissue intended to be excised or otherwise manipulated. The shield member 4 prevents tissue from falling into the space of the working channel 14 between the lesion and the surgeon and the distance indicator 23 provides an immediate visual control of the position and distances of the instruments and tissue portions of interest.

In order to manipulate or excise the lesion, it will be necessary to grasp the tissue in at least one position. This can be done e.g. by placing a traction suture 33, preferably stitching the suture into the tissue portion identified and held by the reference recesses 20 of the first retaining wall 13, by grasping the tissue with a grasper possibly also assisted by the above said reference recesses 20, loop the tissue (e.g. a mushroom shaped polyp) by a snare (not shown) or by attaching other suitable tractioning devices to the tissue. Of course, all these possible tractioning means are transported to the target site through the working channel 14 of the access device 1 and through the tissue access area 16 defined by its first and second retaining walls 13, 26.

After or before having grasped the tissue by the tractioning means the surgical cutter stapler 30, preferably a cutter stapler with a curved staple applying assembly, is introduced through the working channel 14 of the access device 1 up to the target site or in other words up to the depth of the tissue access area 16 defined by the anoscope window aperture 15 and the shield member 4.

As is known, such a cutter stapler comprises two opposite jaws, a proximal jaw provided with a staple driving device and a knife driving device and an opposite distal jaw called anvil which provides a staple forming surface for the rows of staples and a cutting block for the knife exiting from the proximal jaw.

The cutter stapler 30 can be introduced through the working channel 14 of the device 1 either with the jaws in an open position or in a closed position or with the jaws open and the traction means threaded through the aperture defined between the jaws so that the cutter stapler is guided along the traction means.

After having positioned the cutter stapler 30, the suspicious lesion and possibly also some surrounding tissue is placed and pulled between the jaws by means of a controlled traction of the traction means, e.g. by pulling the suture previously applied to the tissue.

The positioning and traction of the lesion between the jaws of the cutter stapler 30 occurs under continuous visual control of the mutual position and distance of tissue access device and surgical instruments thanks to the working channel being kept free from occluding tissue by the shield member and thanks to the distance markings 23 inside the working channel.

After having positioned the tissue between the jaws of the cutter stapler, the latter are approximated to another to clamp the tissue, the staples are fired to fixate the tissue along a staple line which can be performed as a superficial stapled seam or as a full thickness non-circumferential anastomotic stapled seam, and the knife is advanced to excise the tissue specimen, preferably between two adjacent rows of staples.

It is now possible to remove the tissue specimen, e.g. by proximally pulling the traction means, and to visually inspect the site. Should the stapled seam not be satisfactory, it can be completed, e.g. by additional stitches of suture.

Throughout the entire procedure, the slidable shield member 4 is positioned by extracorporeal manual action to adjust and provide the required shape and position of the tissue access area 16 and to retain the surrounding tissue and prevent it from falling into the working channel 14.

As those skilled in the art will have appreciated from the foregoing description, the device 1 and the method for transanal surgery using the device 1 enable surgeons to more simply and consistently treat patients with rectal cancers, benign tumors, polyps, solitary ulcers etc. compared to prior art methodologies. The device and method according to the invention further enable surgeons to perform effective rectal transanal full thickness stapled local excisions using a surgical cutter stapler such as the *curved cutter stapler* CCS-30 by Ethicon Endo-Surgery, Inc.

The device according to the invention enable macrobiopsy histological evaluation to assist in further treatment options and to be curative by itself in some patients.

By way of the above described features and the technical effects thereof, the device according to the invention potentially limit disease recurrence compared to non full thickness excision of lesions.

While the present invention has been illustrated by description of embodiments and while the illustrative embodiments have been described in considerable detail, it is not the intention to restrict or in any way limit the scope of the appended claims to such detail.

## Claims

1. A surgical device (1) for transanally accessing the rectum of a patient, comprising:
- a circular anal dilator (2) having a cylindrical ring wall (6) adapted to be transanally inserted into the rectum in order to provide a transanal access opening therethrough and a connecting portion (8) arranged at a proximal end of the ring wall (6) and adapted to hold the ring wall (6) inside the rectum;
- an anoscope (3) comprising a first retaining wall (13) defining at least partially a working channel (14) and a window aperture (15) providing an access area (16) to the surrounding tissue from inside the working channel (14), said anoscope (3) being received by the ring wall (6) of the anal dilator (2) and having a length such that it protrudes distally from the ring wall (6);
- a shield member (4) comprising a second retaining wall (26) which can be arranged in a manner to partially overlap the window aperture (15) of the anoscope (3) in order to vary the tissue access area (16) thereof,
**characterized in that** the anoscope (3) comprises two recesses (20) formed in two opposite lateral edges (21) of the window aperture (15) of the first retaining wall (13) and adapted to retain and position tissue portions for the application of a purse string suture.

2. A surgical device (1) according to claim 1, wherein said shield member (4) is slidable with respect to said anoscope (3) and said second retaining wall (26) is configured to progressively overlap or close said window aperture (15) in response to a progressive sliding movement of the shield member (4) with respect to the anoscope (3).

3. A surgical device (1) according to claim 2, wherein the window aperture (15) extends in a longitudinal, proximal to distal, direction of the anoscope (3) and the shield member (4) is configured so that the second retaining wall (26) progressively overlaps and closes the window aperture (15) from a proximal side thereof.

4. A surgical device (1) according to any of the preceding claims, wherein the first retaining wall (13) of the anoscope (3) and the second retaining wall (26) of the shield member (4) are slidingly coupled to each other by a longitudinally extending tongue and groove connection (24, 25).

5. A surgical device (1) according to any of the preceding claims, wherein:
- the first retaining wall (13) forms a straight channel having an arched cross-section and defining a longitudinally extending lateral opening forming said anoscope window aperture (15);
- the second retaining wall (26) forms a straight channel having an arched cross-section and being arranged inside said anoscope window aperture (15) so that the first retaining wall (13) and the second retaining wall (26) form a substantially cylindrical tube defining internally said working channel (14).

6. A surgical device (1) according to any of the preceding claims, wherein the anoscope (3) is longitudinally slidable with respect to the circular anal dilator (2) in order to enable adjustment of the distal distance of the tissue access area (16) from the anal verge.

7. A surgical device (1) according to any of the preceding claims, wherein the anoscope (3) comprises a distance indicator (23) adapted to visually indicate the position or distances of tissue portions and/or instruments inside the working channel (14).

8. A surgical device (1) according to the preceding claim, wherein said distance indicator comprises a graduated sequence of marks (23) at a concave internal surface (22) of said first retaining wall (13).

9. A surgical device (1) according to any one of the preceding claims, wherein the two opposite recesses (20) are formed near the distal end (19) of the anoscope (3).

10. A surgical device (1) according to any of the preceding claims, wherein a distal end of the anoscope (3) comprises an arc shaped edge (19) which is inclined with respect to the longitudinal axis (L) of the anoscope (3), thereby facilitating the insertion of the device (1) into the rectum.

11. A surgical device (1) according to any of the preceding claims, wherein the distal end of the shield member (4) comprises an arc shaped edge (28) which is inclined with respect to the longitudinal axis (L) of the shield member (4) to facilitate the insertion of the device (1) into the rectum.

12. A surgical device (1) according to claims 10 and 11, wherein said arc shaped distal edge (28) of the shield member (4) and the lateral edges (21) of the anoscope (3) meet at an obtuse angle in order to prevent tissue damage during insertion of the device (1) and during movement of the shield member (4).

13. A surgical device (1) according to any of the preceding claims, wherein the connecting portion (8) of the circular anal dilator (2) comprises two flat opposite wings (9) protruding laterally outward from the ring wall (6) and forming ring shaped end portions (12) adapted to be sutured to the skin of the buttocks of a patient in order to fixate the position of the circular anal dilator (2) inside the rectum.

14. A surgical device (1) according to any of the preceding claims, comprising an obturator (5) having an elongate body (31) with a smoothly rounded distal tip (32), said obturator (5) being removably insertable through the ring wall (6) of the circular anal dilator and through the working channel (14) defined by the anoscope and the shield member so that the rounded distal tip (32) protrudes distally from the anoscope to facilitate the transanal insertion of the device (1).

15. A surgical device according to any of the preceding claims, wherein the anoscope (3) comprises a handle portion (18) protruding preferably laterally from the proximal end of the anoscope (3) and configured to enable manual rotational and translational positioning of the anoscope (3) inside the ring wall (6) of the anal dilator (2).

## Patentansprüche

1. Chirurgische Vorrichtung (1) für einen transanalen Zugang zum Rektum eines Patienten, umfassend:
einen kreisförmigen Analdilator (2), der eine zylindrische Ringwand (6), die so ausgeführt ist, dass sie transanal in das Rektum eingeführt wird, um hierdurch eine transanale Zugangsöffnung zu schaffen, sowie einen Verbindungsabschnitt (8) aufweist, der an einem proximalen Ende der Ringwand (6) angeordnet ist und so ausgeführt ist, dass er die Ringwand (6) innerhalb des Rektums hält;
ein Anoskop (3), das eine erste Haltewand (13), die wenigstens teilweise einen Arbeitskanal (14) definiert, und eine Fensteröffnung (15) umfasst, die einen Zugangsbereich (16) zu dem umgebenden Gewebe von innerhalb des Arbeitskanals (14) zur Verfügung stellt, wobei das Anoskop (3) von der Ringwand (6) des Analdilators (2) aufgenommen wird und eine solche Länge aufweist, dass es distal aus der Ringwand (6) hervorsteht;
ein Abschirmungselement (4), das eine zweite Haltewand (26) umfasst, die in einer Weise angeordnet werden kann, dass sie die Fensteröffnung (15) des Anoskops (3) teilweise überlappt, um den Gewebezugangsbereich (16) desselben zu variieren,
**dadurch gekennzeichnet, dass** das Anoskop (3) zwei Aussparungen (20) umfasst, die in zwei gegenüberliegenden lateralen Kanten (21) der Fensteröffnung (15) der ersten Haltewand (13) ausgebildet sind und so ausgeführt sind, dass sie Gewebeabschnitte für die Anwendung einer Tabaksbeutelnaht halten und positionieren.

2. Chirurgische Vorrichtung (1) nach Anspruch 1, wobei das Abschirmungselement (4) bezüglich des Anoskops (3) verschiebbar ist und die zweite Haltewand (26) so ausgeführt ist, dass sie in Reaktion auf eine fortschreitende Schiebebewegung des Abschirmungselements (4) bezüglich des Anoskops (3) die Fensteröffnung (15) zunehmend überlappt oder verschließt.

3. Chirurgische Vorrichtung (1) nach Anspruch 2, wobei sich die Fensteröffnung (15) in einer longitudinalen Proximal-zu-distal-Richtung des Anoskops (3) erstreckt und das Abschirmungselement (4) so ausgeführt ist, dass die zweite Haltewand (26) die Fensteröffnung (15) ausgehend von einer proximalen Seite derselben fortschreitend überlappt und verschließt.

4. Chirurgische Vorrichtung (1) nach irgendeinem der vorangehenden Ansprüche, wobei die erste Haltewand (13) des Anoskops (3) und die zweite Haltewand (26) des Abschirmungselements (4) durch eine longitudinal verlaufende Zunge-und-Nut-Verbindung (24, 25) verschiebbar miteinander gekoppelt sind.

5. Chirurgische Vorrichtung (1) nach irgendeinem der vorangehenden Ansprüche, wobei:
die erste Haltewand (13) einen geraden Kanal bildet, der einen gebogenen Querschnitt aufweist und eine longitudinal verlaufende laterale Öffnung definiert, die die Anoskop-Fensteröffnung (15) bildet;
die zweite Haltewand (26) einen geraden Kanal bildet, der einen bogenförmigen Querschnitt aufweist und innerhalb der Anoskop-Fensteröffnung (15) angeordnet ist, so dass die erste Haltewand (13) und die zweite Haltewand (26) ein im Wesentlichen zylindrisches Rohr bilden, das im Inneren den Arbeitskanal (14) definiert.

6. Chirurgische Vorrichtung (1) nach irgendeinem der vorangehenden Ansprüche, wobei das Anoskop (3) bezüglich des kreisförmigen Analdilators (2) longitudinal verschiebbar ist, um ein Einstellen des distalen Abstands des Gewebezugangsbereichs (16) vom Analrand zu ermöglichen.

7. Chirurgische Vorrichtung (1) nach irgendeinem der vorangehenden Ansprüche, wobei das Anoskop (3) einen Abstandsanzeiger (23) umfasst, der so ausgeführt ist, dass er die Position oder die Abstände der Gewebeabschnitte und/oder der Instrumente innerhalb des Arbeitskanals (14) visuell anzeigt.

8. Chirurgische Vorrichtung (1) nach dem vorangehenden Anspruch, wobei der Abstandsanzeiger eine abgestufte Folge von Markierungen (23) an einer konkaven Innenfläche (22) der ersten Haltewand (13) umfasst.

9. Chirurgische Vorrichtung (1) nach irgendeinem der vorangehenden Ansprüche, wobei die zwei gegenüberliegenden Aussparungen (20) nahe dem distalen Ende (19) des Anoskops (3) ausgebildet sind.

10. Chirurgische Vorrichtung (1) nach irgendeinem der vorangehenden Ansprüche, wobei ein distales Ende des Anoskops (3) eine bogenförmige Kante (19) umfasst, die bezüglich der Longitudinalachse (L) des Anoskops (3) geneigt ist, wodurch das Einführen der Vorrichtung (1) in das Rektum erleichtert wird.

11. Chirurgische Vorrichtung (1) nach irgendeinem der vorangehenden Ansprüche, wobei das distale Ende des Abschirmungselements (4) eine bogenförmige Kante (28) umfasst, die bezüglich der Longitudinalachse (L) des Abschirmungselements (4) geneigt ist, um das Einführen der Vorrichtung (1) in das Rektum zu erleichtern.

12. Chirurgische Vorrichtung (1) nach irgendeinem der Ansprüche 10 und 11, wobei sich die bogenförmige distale Kante (28) des Abschirmungselements (4) und die lateralen Kanten (21) des Anoskops (3) in einem stumpfen Winkel treffen, um eine Gewebebeschädigung während des Einführens der Vorrichtung (1) und während der Bewegung des Abschirmungselements (4) zu vermeiden.

13. Chirurgische Vorrichtung (1) nach irgendeinem der vorangehenden Ansprüche, wobei der Verbindungsabschnitt (8) des kreisförmigen Analdilators (2) zwei flache gegenüberliegende Flügel (9) umfasst, die aus der Ringwand (6) lateral nach außen hervorstehen und ringförmige Endabschnitte (12) bilden, die dafür ausgelegt sind, mit der Haut der Gesäßhälften eines Patienten vernäht zu werden, um die Position des kreisförmigen Analdilators (2) innerhalb des Rektums zu fixieren.

14. Chirurgische Vorrichtung (1) nach irgendeinem der vorangehenden Ansprüche, die einen Obturator (5) umfasst, der einen langgestreckten Körper (31) mit einer gleichmäßig abgerundeten distalen Spitze (32) aufweist, wobei der Obturator (5) entnehmbar durch die Ringwand (6) des kreisförmigen Analdilators und durch den Arbeitskanal (14), der von dem Anoskop und dem Abschirmungselement definiert wird, einsetzbar ist, so dass die abgerundete distale Spitze (32) distal aus dem Anoskop hervorsteht, um die transanale Einführung der Vorrichtung (1) zu erleichtern.

15. Chirurgische Vorrichtung nach irgendeinem der vorangehenden Ansprüche, wobei das Anoskop (3) einen Griffabschnitt (18) umfasst, der vorzugsweise lateral aus dem proximalen Ende des Anoskops (3) hervorsteht und so ausgeführt ist, dass er eine manuelle drehende und verschiebende Positionierung des Anoskops (3) innerhalb der Ringwand (6) des Analdilators (2) erlaubt.

## Revendications

1. Dispositif chirurgical (1) pour avoir accès de manière transanale au rectum d'un patient, comprenant :
un dilatateur anal circulaire (2) ayant une paroi annulaire cylindrique (6) adapté pour être inséré de manière transanale dans le rectum afin de fournir une ouverture d'accès transanale à travers celle-ci et une partie de raccordement (8) agencée au niveau d'une extrémité proximale de la paroi annulaire (6) et adaptée pour maintenir la paroi annulaire (6) à l'intérieur du rectum ;
un anuscope (3) comprenant une première paroi de retenue (13) définissant au moins partiellement un canal de travail (14) et une ouverture de fenêtre (15) fournissant une zone d'accès (16) au tissu périphérique depuis l'intérieur du canal de travail (14), ledit anuscope (3) étant reçu par la paroi annulaire (6) du dilatateur anal (2) et ayant une longueur de sorte qu'elle fait saillie de manière distale de la paroi annulaire (6) ;
un élément de protection (4) comprenant une seconde paroi de retenue (26) qui peut être agencée afin de chevaucher partiellement sur l'ouverture de fenêtre (15) de l'anuscope (3) afin de modifier sa zone d'accès au tissu (16),
**caractérisé en ce que** l'anuscope (3) comprend deux évidements (20) formés dans deux bords latéraux opposés (21) de l'ouverture de fenêtre (15) de la première paroi de retenue (13) et adaptés pour retenir et positionner des parties de tissu pour l'application d'une suture en bourse.

2. Dispositif chirurgical (1) selon la revendication 1, dans lequel ledit élément de protection (4) peut coulisser par rapport audit anuscope (3) et ladite seconde paroi de retenue (26) est configurée pour chevaucher progressivement ou fermer ladite ouverture de fenêtre (15) en réponse à un mouvement coulissant progressif de l'élément de protection (4) par rapport à l'anuscope (3).

3. Dispositif chirurgical (1) selon la revendication 2, dans lequel l'ouverture de fenêtre (15) s'étend dans une direction longitudinale proximale à distale de l'anuscope (3) et l'élément de protection (4) est configuré de sorte que la seconde paroi de retenue (26) chevauche progressivement et ferme l'ouverture de fenêtre (15) à partir de son côté proximal.

4. Dispositif chirurgical (1) selon l'une quelconque des revendications précédentes, dans lequel la première paroi de retenue (13) de l'anuscope (3) et la seconde paroi de retenue (26) de l'élément de protection (4) sont couplées de manière coulissante l'une par rapport à l'autre par un raccordement à languette et rainure (24, 25) s'étendant de manière longitudinale.

5. Dispositif chirurgical (1) selon l'une quelconque des revendications précédentes, dans lequel :
la première paroi de retenue (13) forme un canal droit ayant une section transversale arquée et définissant une ouverture latérale s'étendant de manière longitudinale formant ladite ouverture de fenêtre (15) de l'anuscope ;
la seconde paroi de retenue (26) forme un canal droit ayant une section transversale arquée et étant agencée à l'intérieur de ladite ouverture de fenêtre d'ouverture (15) de l'anuscope de sorte que la première paroi de retenue (13) et la seconde paroi de retenue (26) forment un tube sensiblement cylindrique définissant à l'intérieur ledit canal de travail (14).

6. Dispositif chirurgical (1) selon l'une quelconque des revendications précédentes, dans lequel l'anuscope (3) peut coulisser de manière longitudinale par rapport au dilatateur anal circulaire (2) afin de permettre l'ajustement de la distance distale de la zone d'accès au tissu (16) à partir du bord anal.

7. Dispositif chirurgical (1) selon l'une quelconque des revendications précédentes, dans lequel l'anuscope (3) comprend un indicateur de distance (23) adapté pour indiquer visuellement la position ou les distances des parties de tissu et/ou des instruments à l'intérieur du canal de travail (14).

8. Dispositif chirurgical (1) selon l'une quelconque des revendications précédentes, dans lequel l'indicateur de distance comprend une séquence graduée de marques (23) au niveau d'une surface interne concave (22) de ladite première paroi de retenue (13).

9. Dispositif chirurgical (1) selon l'une quelconque des revendications précédentes, dans lequel les deux évidements opposés (20) sont formés à proximité de l'extrémité distale (19) de l'anuscope (3).

10. Dispositif chirurgical (1) selon l'une quelconque des revendications précédentes, dans lequel une extrémité distale de l'anuscope (3) comprend un bord en forme d'arc (19) qui est incliné par rapport à l'axe longitudinal (L) de l'anuscope (3), facilitant ainsi l'insertion du dispositif (1) dans le rectum.

11. Dispositif chirurgical (1) selon l'une quelconque des revendications précédentes, dans lequel l'extrémité distale de l'élément de protection (4) comprend un bord en forme d'arc (28) qui est incliné par rapport à l'axe longitudinal (L) de l'élément de protection (4) afin de faciliter l'insertion du dispositif (1) dans le rectum.

12. Dispositif chirurgical (1) selon les revendications 10 et 11, dans lequel ledit bord distal en forme d'arc (28) de l'élément de protection (4) et les bords latéraux (21) de l'anuscope (3) se rencontrent au niveau d'un angle obtus afin d'empêcher l'endommagement du tissu pendant l'insertion du dispositif (1) et pendant le mouvement de l'élément de protection (4).

13. Dispositif chirurgical (1) selon l'une quelconque des revendications précédentes, dans lequel la partie de raccordement (8) du dilatateur anal circulaire (2) comprend deux ailes plates opposées (9) faisant saillie latéralement vers l'extérieur à partir de la paroi annulaire (6) et formant des parties d'extrémité de forme annulaire (12) adaptées pour être suturées sur la peau des fesses d'un patient afin de fixer la position du dilatateur anal circulaire (2) à l'intérieur du rectum.

14. Dispositif chirurgical (1) selon l'une quelconque des revendications précédentes, comprenant un obturateur (5) ayant un corps allongé (31) avec une pointe distale lisse arrondie (32), ledit obturateur (5) pouvant être inséré de manière amovible à travers la paroi annulaire (6) du dilatateur anal circulaire et à travers le canal de travail (14) défini par l'anuscope et l'élément de protection de sorte que la pointe distale arrondie (32) fait saillie de manière distale à partir de l'anuscope afin de faciliter l'insertion transanale du dispositif (1).

15. Dispositif chirurgical (1) selon l'une quelconque des revendications précédentes, dans lequel l'anuscope (3) comprend une partie de manche (18) faisant saillie de préférence latéralement par rapport à l'extrémité proximale de l'anuscope (3) et configurée pour permettre le positionnement rotatif et translationnel manuel de l'anuscope (3) à l'intérieur de la paroi annulaire (6) du dilatateur anal (2).
